(19) Europäisches Patentamt
European Patent Office
Office européen des brevets

(11) Veröffentlichungsnummer : **0 008 102**
**B1**

(12) **EUROPÄISCHE PATENTSCHRIFT**

(45) Veröffentlichungstag der Patentschrift : **30.09.81**

(21) Anmeldenummer : **79102820.2**

(22) Anmeldetag : **06.08.79**

(51) Int. Cl.³ : **C 07 D498/10**, C 07 D498/20// C08K5/35 ,(C07D498/10, 263/00, 221/00),(C07D498/20, 263/00, 221/00, 221/00)

(54) **Verfahren zur Herstellung von Azaspirodecanen.**

(30) Priorität : **10.08.78 DE 2834962**

(43) Veröffentlichungstag der Anmeldung : **20.02.80 (Patentblatt 80/04)**

(45) Bekanntmachung des Hinweises auf die Patenterteilung : **30.09.81 Patentblatt 81/39**

(84) Benannte Vertragsstaaten : **CH DE FR GB IT NL SE**

(56) Entgegenhaltungen :

**CHEMISTRY AND INDUSTRY, Band 38, 21-09-1968, Seite 1276, London, G.B.**
**J.W. DUCKER : « The Ritter reaction between cyclohexanone and cyanohydrins »**

(73) Patentinhaber : **HOECHST AKTIENGESELLSCHAFT**
**Postfach 80 03 20**
**D-6230 Frankfurt/Main 80 (DE)**

(72) Erfinder : **Wiezer, Hartmut, Dr.**
**Hans-Fischer-Strasse 6**
**D-8906 Gersthofen (DE)**
Erfinder : **Korbanka, Helmut, Dr.**
**Birkenstrasse 24**
**D-8901 Adelsried (DE)**
Erfinder : **Mayer, Norbert, Dr.**
**Ziegelgrundweg 17**
**D-8901 Gablingen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 008 102 B1

Imprimerie Jouve, 17, rue du Louvre, 75001 Paris, France

# Verfahren zur Herstellung von Azaspirodecanen

1-Oxa-diaza-oxo-spirodecane sind aus DE-A-2 634 957 und DE-A-2 606 026 bekannte Substanzen, die hervorragend zum Stabilisieren von synthetischen Polymeren geeignet sind.

Es handelt sich um Verbindungen der allgemeinen Formel (I)

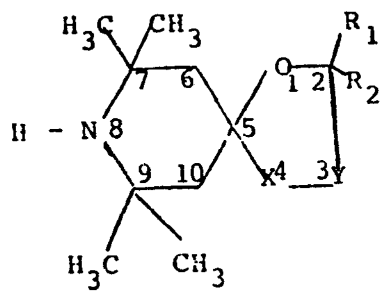

(I),

in welcher

X und Y verschieden sind und die Bedeutung von -CO oder -NH haben,

$R^1$ für Wasserstoff, $C_1$- bis $C_{30}$-Alkyl, vorzugsweise $C_1$- bis $C_{18}$-Alkyl, insbesondere $C_1$- bis $C_5$-Alkyl, für eine unsubstituierte oder durch Chlor oder $C_1$- bis $C_4$-Alkyl substituierte Phenyl- oder Naphthylgruppe, vorzugsweise die erstgenante, oder für eine unsubstituierte oder durch $C_1$- bis $C_4$-Alkyl substituierte $C_7$- bis $C_{12}$-Phenylalkylgruppe, vorzugsweise für eine Benzylgruppe, steht

$R^2$ Wasserstoff, $C_1$- bis $C_{30}$-Alkyl, vorzugsweise $C_1$- bis $C_{18}$-Alkyl, insbesondere $C_1$- bis $C_{13}$-Alkyl, eine unsubstituierte Phenyl- oder Naphthylgruppe, vorzugsweise eine Phenylgruppe, eine unsubstituierte oder durch $C_1$- bis $C_4$-Alkyl substituierte $C_7$- bis $C_{12}$-Phenylalkylgruppe, vorzugsweise eine Benzylgruppe ist, oder

$R^1$ und $R^2$ zusammen mit dem an sie gebundenen Kohlenstoffatom die Bedeutung einer unsubstituierten oder durch bis zu vier $C_1$- bis $C_4$-Alkylgruppen, vorzugsweise Methylgruppen substituierten $C_5$- bis $C_{18}$-Cycloalkylgruppe, vorzugsweise $C_5$- bis $C_{12}$-Cycloalkylgruppe oder einer Gruppe der Formel

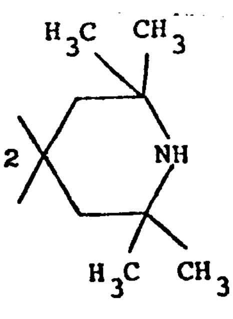

haben.

Verfahren zur Herstellung der genannten 1-Oxa-diaza-oxo-spiro-decane sind in den obengenannten Druckschriften beschrieben (siehe auch DE-A-1 770 791 und DE-A-2 109 333). Man geht dabei entweder vom Piperidoncyanhydrin aus, welches zunächst in die Carbamoylverbindung überführt werden muß, worauf man diese mit Aldehyden oder Ketonen umsetzt, oder man läßt das Piperidon mit einem α-Hydroxycarbonsäureamid, reagieren, welches seinerseits aus einem Aldehyd oder Keton durch Anlagerung von HCN und Verseifung des Cyanhydrins synthetisierbar ist. Nachteile dieser Verfahren sind mehrere umständliche Reaktionsstufen, die zum Teil mit unbefriedigenden Ausbeuten verlaufen und die Notwendigkeit, die Zwischenprodukte isolieren zu müssen, so daß die Gesamtausbeuten im allgemeinen bei nur 35 bis 65 % liegen.

Es sind auch noch Verfahren bekannt, nach welchen aliphatische oder cycloaliphatische, nicht basische Cyanhydrine mit Ketonen derselben Provenienz direkt umgesetzt werden können, wobei in Gegenwart großer Mengen Schwefelsäure, die gleichzeitig als Lösungsmittel und Katalysator dient, gearbeitet wird. Man kann nach diesem Verfahren z.B. aus Cyclohexanoncyanhydrin bzw. Acetoncyanhydrin und Cyclohexanon Oxazolidinone herstellen [Ducker, Chem. and Ind. *38* (1968), S. 1276]. In der JA-PS 4 318 898 wird schließlich vorgeschlagen, in gleicher Weise in nichtflüchtigen Mineralsäuren wie Schwefelsäure oder Polyphosphorsäure unter Zusatz von Lösungsmitteln, z.B. Diethyläther oder Toluol, oder in einem großen Überschuß an Carbonylverbindung umzusetzen. Will man nach diesen Methoden jedoch 1-Oxa-diaza-oxo-spirodecane herstellen, so läuft im letztgenannten Falle die gewünschte Reaktion überhaupt nicht ab, während bei der erstgenannten Arbeitsweise ein noch größerer Schwefelsäureüberschuß notwendig ist, um die basischen, während der Reaktion als Schwefelsäuresalze vorliegenden Ausgangsmaterialien in Lösung zu halten. Da zur Isolierung der Verfahrensprodukte die gesamte Schwefelsäure neutralisiert werden muß, fallen unvertretbar hohe Mengen an umweltbelastenden Salzen an, so daß sich auch dieses Verfahren nicht zur Herstellung von 1-Oxa-diaza-oxo-spirodecanen im technischen Rahmen eignet.

Der vorliegenden Erfindung lag daher die Aufgabe zugrunde, ein technisch realisierbares, wirtschaftliches Verfahren für die Herstellung der Azaspirodecane zu entwickeln, das die aufgezeigten Nachteile nicht besitzt.

Es wurde nun gefunden, daß man 1-Oxo-diaza-oxo-spirodecane überraschenderweise unter Umgehung der aufwendigen α-Hydroxycarbonsäureamidstufe in Anlehnung an die letztgenannten Verfahren erhalten kann, wenn man Piperidoncyanhydrin mit Aldehyd oder Keton oder Aldehyd- oder Ketoncyanhydrin mit Piperidon direkt umsetzt und dabei bestimmte Bedingungen einhält.

Die vorliegende Erfindung betrifft demzufolge ein Verfahren zur Herstellung von 1-Oxa-diaza-oxo-spirodecanen der Formel (I)

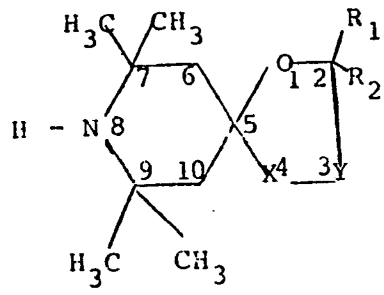

(I),

in der X, Y und $R^1$ und $R^2$ die weiter vorne angegebene Bedeutung haben in dem man a) im Falle der Herstellung von Verbindungen mit X = CO und Y = NH von 2,2,6,6-Tetramethylpiperidon-4-cyanhydrin der Formel (II)

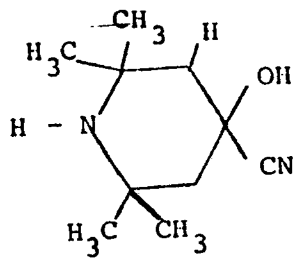

(II),

und einem Aldehyd oder Keton der Formel (III) ausgeht

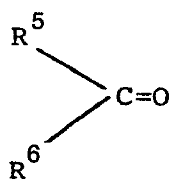

(III)

oder im Falle der Herstellung von Verbindungen mit X = NH und Y = CO von 2,2,6,6-Tetramethylpiperidon-4 der Formel (IV),

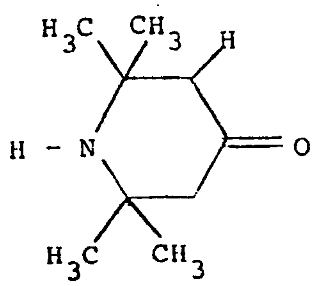

(IV)

und einem Aldehyd- oder Ketoncyanhydrin der Formel (V) ausgeht

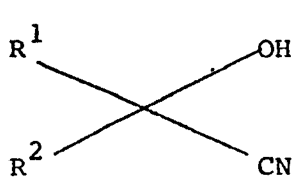

(V)

wobei die Reste $R^1$ und $R^2$ die angegebene Bedeutung haben, welcher dadurch gekennzeichnet ist, daß man die entsprechenden Komponenten dircht in Gegenwart einer bei Raumtemperatur flüssigen aliphatischen Carbonsäure als Lösungsmittel, bei 20 bis 120 °C in Anwesenheit von $H_2SO_4$ oder Halogenwasserstoff umsetzt, wobei die Carbonylverbindungen (III) bzw. (IV) in der 1- bis 3-fachen molaren Menge, bezogen auf die umzusetzenden Cyanhydrine (II) bzw. (V) eingesetzt werden, und die Protonensäuremenge so bemessen wird, daß das Piperidoncyanhydrin (II) bzw. das Piperidon (IV) als Salze vorliegt und zusätzlich die 0,5- bis 2-fach molare Menge, bezogen auf umzusetzendes Cyanhydrin vorhanden ist, worauf man aus dem gebildeten Protonensäuresalz des gewünschten Azaspirodecans die freie Base durch Behandeln mit einer stärkeren Base in Freiheit setzt.

Nach der erfindungsgemäßen Arbeitsweise werden reine Azaspirodecane in guten Ausbeuten erhalten, was nicht zu erwarten war. Zwar wird in Survey of organic Synthesis Vol 2, John Wiley & Sons 1977 S. 437 die Durchführung der Ritter-Reaktion eines Olefins mit Nitrilen in $H_2SO_4$/Eisessig beschrieben; jedoch werden dort nur Komponenten eingeseht, die keine weiteren funktionellen Gruppen besitzen und daher Anlaß zu Nebenreaktionen geben können. Das erfindungsgemäße Verfahren benutzt dagegen Hydroxynitrile als Ausgangsmaterialien von denen bekannt ist (siehe z.B. Zagorevskii, J. Org. Chem. USSR, 1 (1966), 1 066-1 030 oder Bucherer J. prakt. Chem. 140 (1934) S 129-171), daß sie bei Anwesenheit von $H_2SO_4$ zu Nebenreaktionen in erheblichem Ausmaß führen. Aufgrund dieses Standes der Technik konnte somit nicht erwartet werden, daß nach der erfindungsgemäßen Arbeitsweise Azospirodecane in guten Ausbeuten erhalten werden können. Man mußte vielmehr damit rechnen, daß die Umsetzung auch in Gegenwart niedriger aliphatischer Carbonsäuren nicht wie gewünscht ablaufen würde, nachdem — aufgrund eigener Versuchsergebnisse — Piperidoncyanhydrine unter denselben Reaktionsbedingungen in einer Ritter-Reaktion mit sich selbst reagieren können. Daß Nebenprodukte, nur in sehr untergeordnetem Maße entstehen muß als außerordentlich überraschend angesehen werden.

Es war des weiteren überraschend, daß das Gelingen der Umsetzung augenscheinlich nicht davon abhängt, daß das Ausgangsmaterial in irgendeinem organischen Lösungsmittel gelöst vorliegt, sondern vielmehr von der Art dieses Lösungsmittels. So läuft die gewünschte Umsetzung beispielsweise in Dimethylformamid oder Ethylenglykol, in denen sich die Ausgangsstofe und zum Teil auch die Endprodukte gut lösen, nicht ab.

Piperidoncyanhydrin (welches auch in situ aus Piperidon und Alkalimetallcyanid in einer Vorreaktion in demselben Lösungsmittel hergestellt worden sein kann) und Aldehyd oder Keton bzw. Aldehyd- oder Ketoncyanhydrin und Piperidon werden erfindungsgemäß in Gegenwart der als Lösungsmittel dienenden, bei Raumtemperatur

flüssigen, aliphatischen Carbonsäure miteinander umgesetzt, wobei $H_2SO_4$ oder Halogenwasserstoff als Katalysator anwesend sein muß. Geeignete aliphatische Carbonsäuren sind niedrige Fettsäuren, bevorzugt ist Essigsäure. Die Menge beträgt das 1- bis 6-, vorzugsweise das 1,1- bis 3-fache, bezogen auf das Gewicht der Reaktionskomponenten. Als Halogenwasserstoff eignet sich besonders Chlorwasserstoff. Die Protonensäure muß in einer solchen Menge zugesetzt werden, daß die basischen Reaktionspartner als Salze derselben vorliegen und zusätzlich die 0,5- bis 2,0-fache, vorzugsweise die 0,7- bis 1,5-fache molare Menge Protonensäure, bezogen auf eingesetztes Cyanhydrin, als Katalysator vorhanden ist. Bei chlorwasserstoff ist die Umsetzung vorteilhaft unter Druck durchzuführen.

Die Reaktionstemperatur kann zwischen 20 und 120, vorzugsweise zwischen 20 und 90 °C, beim Einsatz von Aldehyden der Formel (III) insbesondere zwischen 20 und 60 °C, bei Ketonen der Formel (III) bzw. (IV) insbesondere zwischen 50 und 90 °C liegen. Die Reaktionszeit beträgt 5 bis 60, vorzugsweise 10 bis 40 und insbesondere 15 bis 30 Stunden. Nach beendeter Umsetzung liegen die gewünschten Azaspirodecane in Form ihrer Protonensäuresalze vor. Einige fallen bereits kristallin an und können durch Abfiltrieren gewonnen werden. Bleiben die Salze in Lösung, so kann man sie durch Ausfällen mit Nichtlösern wie z.B. Ether oder Aceton isolieren. Es ist auch möglich, die Reaktionslösungen einzuengen und dann aufzuarbeiten.

Die freien Basen werden aus den Salzen durch Neutralisation mit stärkeren Basen wie Ammoniak, Aminbassen, Alkalimetallhydroxiden, Alkalimetallcarbonaten oder Alkalimetallalkoholen erhalten.

Die folgenden Beispiele dienen der weiteren Erläuterung des Verfahrens.

Beispiel 1

2,2,7,7,9,9-Hexamethyl-1-oxa-4,8-diaza-3-oxo-spiro-[4,5]-decan

In einem 1-l-Glaskolben wird ein Gemisch aus 500 g Eisessig, 155 g 2,2,6,6-Tetramethylpiperidon-4 (1 Mol), 85 g Acetoncyanhydrin (1 Mol) und 200 g $H_2SO_4$ konz. (als letztes zufügen) 25 Stunden bei 70 °C gerührt. Nach dem Erkalten wird abgenutscht und der Feststoff zweimal mit je 130 ml Aceton eisessigfrei gewaschen. Es fallen 234 g = 69 % d.Th. des $H_2SO_4$-Salzes der gewünschten Verbindung an.

Beispiel 2

2,2,7,7,9,9-Hexamethyl-1-oxa-3,8-diaza-4-oxo-spiro-[4,5]-decan

Analog Beispiel 1 werden in 500 g Eisessig 116 g Aceton (2 Mol), 182 g 2,2,6,6-Tetramethyl-4-hydroxy-4-cyanopiperidin (1 Mol) und 200 g $H_2SO_4$ konz. umgesetzt. Es fallen 254 g = 75 % d.Th. $H_2SO_4$-Salz der gewünschten Verbindung an.

Beispiel 3

2-(2,4,4-Trimethyl-hexyl)-7,7,9,9-tetramethyl-1-oxa-3,8-diaza-4-oxo-spiro-[4,5]-decan

In einer 2-l-Rührapparatur werden in 600 g Eisessig 182 g 2,2,6,6-Tetramethyl-4-hydroxy-4-cyanopiperidin (1 Mol), 284 g iso-Nonylaldehyd der Firma Ruhrchemie AG mit einem Gehalt von > 90 % 3,5,5-Trimethylhexanal-(1) (Rest isomere $C_9$-Aldehyde) (2 Mol) und 200 g $H_2SO_4$ konz. 25 Std. bei 60 °C gerührt. Nach dem Erkalten wird im Vakuum die Hauptmenge des Lösungsmittels abdestilliert. Der Rückstand wird mit 500 ml Aceton verrührt und abgenutscht, wobei 292 g = 69 % d.Th. des $H_2SO_4$-Salzes der gewünschten Verbindung anfallen.

Beispiel 4

2,2,4,4-Tetramethyl-3,14-diaza-7-oxa-15-oxo-dispiro-[5,1,5,2]-pentadecan

In einer 1-l-Rührapparatur werden 182 g 2,2,6,6-Tetramethyl-4-hydroxy-4-cyano-piperidin (1 Mol), 200 g Cyclohexanon (2 Mol) 500 g Eisessig und 200 g $H_2SO_4$ konz. 26 Stunden bei 60 °C gerührt. Nach dem Erkalten wird abgenutscht und zweimal mit 130 ml Aceton nachgewaschen, wobei 278 g = 75 % d.Th. $H_2SO_4$-Salz der gewünschten Verbindung anfallen.

Beispiel 5

2,2,4,4,10,10,12,12-Octamethyl-3,14-diaza-7-oxa-15-oxodispiro-[5,1,5,2,]-pentadecan

In einer 250-ml-Rührapparatur werden 14,6 g 2,2,6,6-Tetramethyl-4-hydroxy-4-cyanopiperidin (0,08 Mol), 18,8 g 3,3,5,5-Tetramethyl-cyclohexanon (0,12 Mol) und 15,9 g $H_2SO_4$ konz. in 120 ml Eisessig 23 Stunden auf 70 °C unter Rühren erhitzt. Nach dem Abkühlen wird abgenutscht, mit Aceton gewaschen und getrocknet, wobei 20 g = 56 % d.Th. $H_2SO_4$-Salz der gewünschten Verbindung anfallen.

Beispiel 6

2,2,4,4,10,12-Hexamethyl-3,14-diaza-7-oxa-15-oxo-dispiro-[5,1,5,2]-pentadecan

In einer 250-ml-Rührapparatur werden 18,2 g 2,2,6,6-Tetramethyl-4-hydroxy-4-cyanopiperidin (0,1 Mol), 12,9 g 3,5-Dimethyl-cyclohexanon (0,1 Mol), 150 ml Eisessig und 19,6 g $H_2SO_4$ konz. 43 Stunden auf 70 °C erhitzt. Nach dem Abkühlen werden 23 g = 74,5 % d.Th. $H_2SO_4$-Salz der gewünschten Verbindung abgenutscht.

Beispiel 7

2,7,7,9,9-Pentamethyl-2-octadecyl-1-oxa-3,8-diaza-4-oxo-spiro-[4,5]-decan

Die Darstellung erfolgt analog Beispiel 6 aus 18,2 g 2,2,6,6-Tetramethyl-4-hydroxy-4-cyanopiperidin (0,1 Mol), und 44,4 g Eicosanon-2 (0,15 Mol), wobei 42 g = 73 % d.Th. des $H_2SO_4$-

Salzes der gewünschten Verbindung anfallen.

Beispiel 8

2,2,4,4-Tetramethyl-3,20-diaza-7-oxa-21-oxo-dispiro-[5,1,11,2]-heneicosan

In einer 1-1-Rührapparatur werden 100 g 2,2,6,6-Tetramethyl-4-hydroxy-4-cyanopiperidin (0,55 Mol), 120 g Cyclododecanon (0,66 Mol), 300 g Eisessig und 94,5 g $H_2SO_4$ konz. 25 Stunden bei 80 °C gerührt, wobei nach dem Erkalten 207 g = 82 % d.Th. des $H_2SO_4$-Salzes der gewünschten Verbindung anfallen.

Zur Isolierung der freien Base werden 100 g des $H_2SO_4$-Salzes in 500 ml Ethanol, in dem 15 g Natrium als Alkoholat enthalten sind, unter Rühren 2 Stunden gekocht. Der Alkohol wird abdestilliert, der Rückstand in 300 ml Wasser verrührt und der Feststoff sodann abgenutscht.

Ausbeute : 68,5 g = 88 % d.Th., bezogen auf eingesetztes Salz. Fp. 226-228 °C.

Beispiel 9

Die Verbindung des Beispiels 8 kann auch in folgender Weise hergestellt werden :

77,5 g 2,2,6,6-Tetramethylpiperidon (0,5 Mol) werden in 300 g Eisessig gelöst, worauf man 33 g Kaliumcyanid (0,5 Mol) zugibt. Dabei steigt die Temperatur auf ca. 50 °C an. Es wird 3 Stunden bei 50 °C nachgerührt und nach dem Abkühlen auf Raumtemperatur mit Stickstoff überschüssiger Cyanwasserstoff ausgeblasen. Dann fügt man 100 g Cyclododecanon (0,55 Mol) und 150 g $H_2SO_4$ konz. zu und rührt 30 Stunden bei 70 °C. Nun wird der Eisessig im leichten Vakuum abdestilliert und sodann der Rückstand mit 1 Liter Wasser aufgeschlämmt. Nun gibt man 240 g 50 % ige NaOH zu und rührt 1/2 Stunde bei 80 °C. Nach dem Abnutschen wird mit Wasser und Aceton gewaschen, wobei 115 g = 63 % d.Th. des gewünschten Produktes in Form der freien Base erhalten werden.

Beispiel 10

2,7,7,9,9-Pentamethyl-2-undecyl-1-oxa-3,8-diaza-4-oxo-spiro-[4,5]-decan

Man erhält diese Verbindung analog Beispiel 6 aus 18,2 g 2,2,6,6-Tetramethyl-4-hydroxy-4-cyanopiperidin (0,1 Mol) und 25 g Tridecanon-2 (0,13 Mol). Es fallen 26,2 g = 54,5 % d.Th. $H_2SO_4$-Salz der gewünschten Verbindung an.

Beispiel 11

2,2-Dipentyl-7,7,9,9-tetramethyl-1-oxa-3,8-diaza-4-oxo-spiro-[4,5]-decan

Die Herstellung erfolgt analog Beispiel 6 aus 18,2 g 2,2,6,6-Tetramethyl-4-hydroxy-4-cyano-piperidin (0,1 Mol) und 34 g Undecanon-6 (0,2 Mol), wobei 31,8 g = 71 % d.Th. des $H_2SO_4$-Salzes der gewünschten Verbindung anfallen.

Beispiel 12

7,7,9,9-Tetramethyl-2,2',4'-dichlorphenyl-1-oxa-3,8-diaza-4-oxo-spiro-[4,5]-decan

18,2 g 2,2,6,6-Tetramethyl-4-hydroxy-4-cyanopiperidin (0,1 Mol), 31 g 2,4-Dichlorbenzaldehyd (0,18 Mol) und 20 g konz. $H_2SO_4$ werden in 100 g Eisessig 47 Stunden bei 60 °C gerührt. Dann wird abgenutscht, mit Aceton gewaschen und getrocknet, wobei 27 g = 58,1 % d.Th. des $H_2SO_4$-Salzes der gewünschten Verbindung anfallen.

Beispiel 13

2,2,4,4-Tetramethyl-7-oxa-14-oxo-3,15-diaza-dispiro-[5,1,5,2]-pentadecan

Die Darstellung des $H_2SO_4$-Salzes der gewünschten Verbindung erfolgt durch Umsetzen von 31 g 2,2,6,6-Tetramethylpiperidon-4 (0,2 Mol) mit 25 g Cyclohexanoncyanhydrin (0,2 Mol) in Gegenwart von 40 g konz. $H_2SO_4$ und 150 g Eisessig bei 70 °C. Reaktionszeit 40 Stunden. Ausbeute an Schwefelsäuresalz 41,5 g = 61,4 % d.Th.

Beispiel 14

7,7,9,9-Tetramethyl-2,2-dibenzyl-1-oxa-3,8-diaza-4-oxo-spiro-[4,5]-decan

18,2 g 2,2,6,6-Tetramethyl-4-hydroxy-4-cyanopiperidin (0,1 Mol), 40 g Dibenzylketon (0,19 Mol) und 20 g $H_2SO_4$ konz. werden in 100 g Eisessig gegeben und 48 Stunden bei 75 °C gerührt. Anschließend wird eingeengt, in Aceton aufgeschlämmt und abgenutscht. Es fallen 29,9 g = 61,0 % d.Th. in Form des Schwefelsäuresalzes an.

**Ansprüche**

1. Verfahren zur Herstellung von Azaspirodecanen der allgemeinen Formel (I)

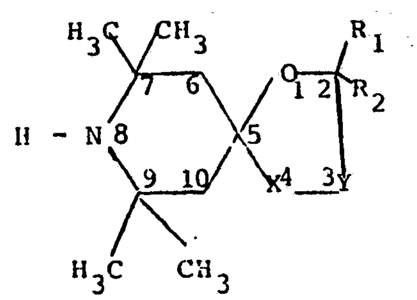

(I),

worin

X und Y verschieden sind und die Bedeutung von -CO oder -NH haben,

$R^1$ und $R^2$ gleich oder verschieden sind und für Wasserstoff, $C_1$- bis $C_{30}$-Alkyl, für eine unsubstituierte oder durch Chlor oder $C_1$- bis $C_4$-Alkyl substituierte Phenyl- oder Naphthylgruppe oder für eine unsubstituierte oder durch $C_1$- bis $C_4$-alkylsubstituierte $C_7$- bis $C_{12}$-Phenylalkylgruppe stehen, oder

$R^1$ und $R^2$ zusammen mit dem an sie gebundenen Kohlenstoffatom die Bedeutung einer unsubstituierten oder durch bis zu vier $C_1$- bis $C_4$-Alkylgruppen substituierten $C_5$- bis $C_{18}$-Cycloalkylgruppe oder einer Gruppe der Formel

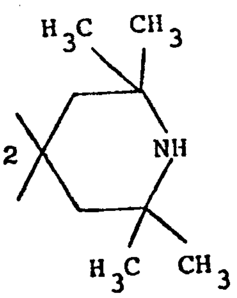

haben in dem man

a) im Falle der Herstellung von Verbindungen mit X = CO und Y = NH von 2,2,6,6-Tetramethylpiperidon-4-cyanhydrin der Formel (II)

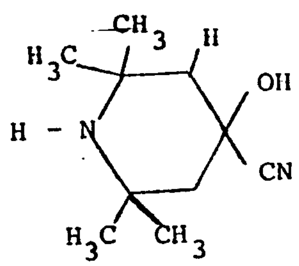 (II),

und einem Aldehyd oder Keton der Formel (III) ausgeht

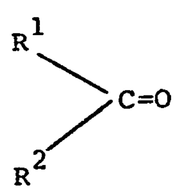 (III)

oder

b) im Falle der Herstellung von Verbindungen mit X = NH und Y = CO von 2,2,6,6-Tetramethylpiperidon-4 der Formel (IV)

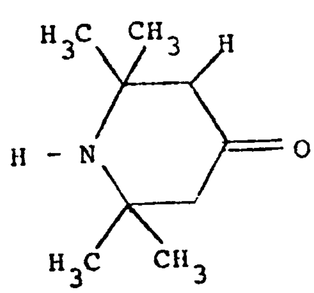 (IV)

und einem Aldehyd- oder Ketoncyanhydrin der Formel (V) ausgeht

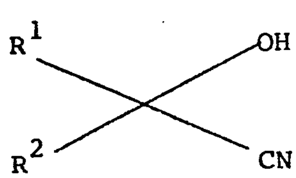 (V)

dadurch gekennzeichnet, daß man die entsprechenden Komponenten dircht in Gegenwart einer bei Raumtemperatur flüssigen, aliphatischen. Carbonsäure als Lösungsmittel, bei 20 bis 120 °C in Anwesenheit von $H_2SO_4$ oder Halogenwasserstoff umsetzt, wobei die Carbonylverbindungen (III) bis (IV) in der 1- bis 3-fachen molaren Menge, bezogen auf die umzusetzenden Cyanhydrine (II) bzw. (V) eingesetzt werden, und die Protonensäuremenge so bemessen wird, daß das Piperidoncyanhydrin (II) bzw. das Piperidon (IV) als Salze vorliegen und zusätzlich die 0,5- bis 2-fach molare Menge, bezogen auf umzusetzendes Cyanhydrin vorhanden ist, worauf man aus dem gebildeten Protonensäuresalz des gewünschten Azaspirodecans die freie Base durch Behandeln mit einer stärkeren Base in Freiheit setzt.

2. Verfahren nach Anspruch 1, dadurch gekennzeichnet, daß die als Lösungsmittel dienende aliphatische Carbonsäure Essigsäure ist.

**Claims**

1. A process for the manufacture of azaspirodecanes of the formula (I)

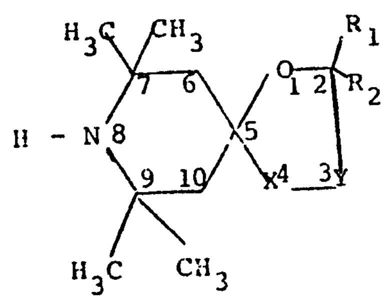 (I),

in which

X and Y being different from one another, each are -CO or -NH ;

$R^1$ and $R^2$ being identical or different, each are hydrogen, $C_1$-$C_{30}$ alkyl, a phenyl or naphthyl group being unsubstituted of substituted by chlorine or $C_1$-$C_4$-alkyl, or a $C_7$-$C_{12}$-phenylalkyl group being unsubstituted or substituted by $C_1$-$C_4$-alkyl ; or

$R^1$ and $R^2$, together with the carbon atom linked to them are a $C_5$-$C_{18}$-cycloalkyl group being unsubstituted or substituted by up to four $C_1C_4$-alkyl groups, or a group of the formula

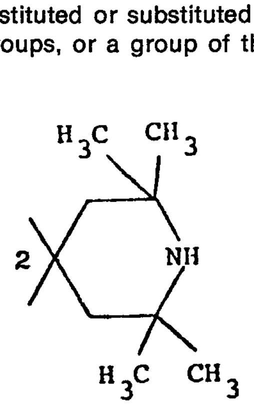

in which proçess a) a 2,2,6,6-tetramethyl-piperidone-4-cyanohydrine of the formula (II)

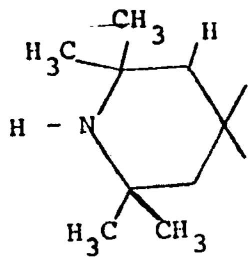

(II),

and an aldehyde or ketone of the formula (III)

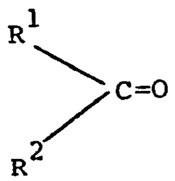

(III)

are used as starting compounds when compounds wherein X = CO and Y = NH shall be produced or b) a 2,2,6,6-tetramethyl-piperidone-4 of the formula (IV)

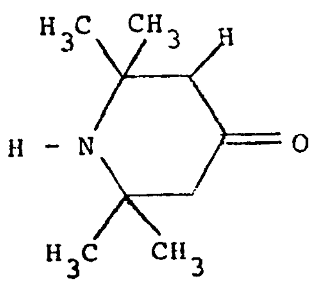

(IV)

and an aldehyde- or ketone-cyanohydrine of the formula (V)

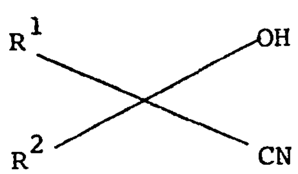

(V)

are used as starting compounds, when compounds wherein X = NH and Y = CO, shall be produced, which comprises reacting the corresponding components directly in the presence of an aliphatic carboxylic acid liquid at room temperature, as solvent, at a temperature of from 20 to 120 °C and in the presence of $H_2SO_4$ or hydrogen halide, the carbonyl compounds (III) and (IV), respectively, being used in a 1- to 3-fold molar amount relative to the cyanohydrines (II) and (V), respectively to be converted, and the amount of protonic acid being chosen in such a manner that the piperidone-cyanohydrine (II) and the piperidone (IV), respectively, are present as salts, while the 0.5 to 2-fold molar amount, relative to the cyanohydrine to be converted, is present in addition, and by subsequently liberating the free base from the protonic acid salt of the intended azaspirodecane so obtained by treatment with a stronger base.

2. The process as claimed in Claim 1, wherein the aliphatic carboxylic acid serving as solvent is acetic acid.

**Revendications**

1. Procédé de préparation d'azaspirodécanes répondant à la formule générale (I)

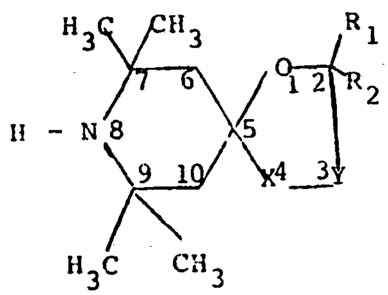

(I),

dans laquelle

X et Y sont différents l'un de l'autre et représentent -CO- ou -NH-,

$R^1$ et $R^2$ sont identiques ou différents et représentent chacun un atome d'hydrogène, un radical alkyle en $C_1$-$C_{30}$, un radical phényle ou naphtyle non substitué ou porteur d'un atome de chlore ou d'un alkyle en $C_1$-$C_4$, ou un radical phénylalkyle en $C_7$-$C_{12}$ non substitué ou porteur d'un alkyle en $C_1$-$C_4$, ou encore

$R^1$ et $R^2$ forment ensemble, et avec l'atome de carbone auquel ils sont liés, un radical cycloalkyle en $C_5$-$C_{18}$ non substitué ou porteur d'un à quatre alkyles en $C_1$-$C_4$, ou un radical de formule

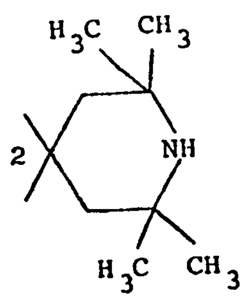

selon lequel

a) lorsqu'on veut préparer des composés pour lesquels X désigne -CO- et Y désigne -NH- on part de la cyanhydrine de la tétraméthyl-2,2,6,6 pipéridone-4 de formule (II)

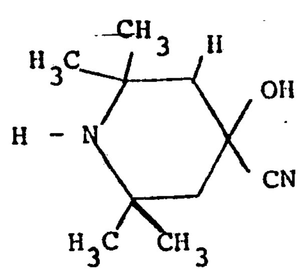

(II)

et d'un aldéhyde ou d'une cétone de formule (III)

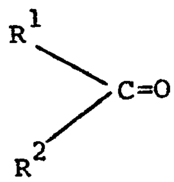

(III)

b) lorsqu'on veut préparer des composés pour lesquels X désigne -NH- et Y désigne -CO- on part de la tétraméthyl-2,2,6,6 pipéridone-4 de formule (IV)

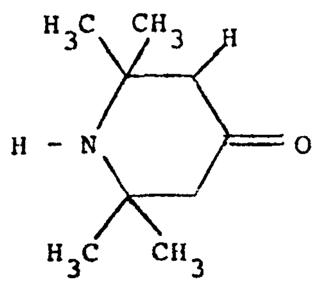

(IV)

et d'une aldéhyde-cyanhydrine ou d'une cétone-cyanhydrine de formule (V)

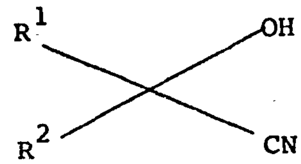

(V)

procédé caractérisé en ce qu'on fait réagir les composantes correspondantes directement dans un acide carboxylique aliphatique liquide à la température ambiante, qui sert de solvant, à une température de 20 à 120 °C, en présence de $H_2SO_4$ ou d'un acide halohydrique, les composés carbonyliques (III) ou (IV) étant mis en jeu en une quantité triple de la quantité molaire par rapport aux cyanhydrines (II) ou (V) à faire réagir et la quantité d'acide protonique étant ajustée de telle façon que la pipéridone-cyanhydrine (II) ou la pipéridone (IV) soit sous la forme de sel et qu'il y en ait un supplément représentant de 0,5 à 2 fois la quantité molaire par rapport à la cyanhydrine à faire réagir, après quoi, du sel formé par l'acide protonique et l'azaspirodécane voulu, on libère la base par traitement avec une base plus forte.

2. Procédé selon la revendication 1, caractérisé en ce que l'acide carboxylique aliphatique servant de solvant est l'acide acétique.